# EUROPEAN PATENT SPECIFICATION

(11) **EP 1 511 494 B1**
(45) Date of publication and mention of the grant of the patent: **19.08.2020**
(21) Application number: 03726062.7
(22) Date of filing: 14.03.2003
(51) Int. Cl.: A61K 9/00, A61K 47/10, A61K 47/14, A61K 47/32, A61K 31/56, A61P 25/24

(54) **ANDROGEN PHARMACEUTICAL COMPOSITION AND METHOD FOR TREATING DEPRESSION**
PHARMAZEUTISCHE ANDROGEN-ZUSAMMENSETZUNG UND VERFAHREN ZUR BEHANDLUNG VON DEPRESSIONEN
COMPOSITION PHARMACEUTIQUE ANDROGÈNE ET PROCÉDÉ POUR LE TRAITEMENT DE LA DÉPRESSION

(30) Priority: 15.03.2002 US 98232; 21.05.2002 US 153468
(43) Date of publication of application: 09.03.2005
(73) Proprietor: Unimed Pharmaceuticals, LLC, Marietta, GA 30062 (US); Besins Healthcare Luxembourg SARL, 2668 Luxembourg (LU)
(72) Inventor: DUDLEY, Robert, E., Kenilworth, IL 60043 (US); KOTTAYIL, George, S., Long Grove, IL 60047 (US); PALATCHI, Olivier, 94240 L'Hay Les Rosses (FR)
(74) Representative: Nederlandsch Octrooibureau
(86) International application number: PCT/US2003/007910
(87) International publication number: WO 2004/091631

(56) References cited:
- WO-A1-02/17926
- WO-A1-02/17967
- WO-A1-96/27372
- WO-A1-98/34621
- WO-A1-99/24041
- US-A- 5 880 117
- WANG C ET AL: "TRANSDERMAL TESTOSTERONE GEL IMPROVES SEXUAL FUNCTION, MOOD, MUSCLE STRENGTH, AND BODY COMPOSITION PARAMETERS IN HYPOGONADAL MEN" JOURNAL OF CLINICAL ENDOCRINOLOGY AND METABOLISM, THE ENDOCRINE SOCIETY, US LNKD- DOI:10.1210/JC.85.8.2839, vol. 85, no. 8, 1 August 2000 (2000-08-01) , pages 2839-2853, XP001087585 ISSN: 0021-972X
- Cláudio De Novaes Soares ET AL: "Efficacy of Estradiol for the Treatment of Depressive Disorders in Perimenopausal Women: A Double-blind, Randomized, Placebo-Controlled Trial", Archives of General Psychiatry, 1 June 2001 (2001-06-01), pages 529-534, XP055442570, United States DOI: 10.1001/archpsyc.58.6.529 Retrieved from the Internet: URL:https://jamanetwork.com/journals/jamap sychiatry/articlepdf/481788/yoa20238.pdf [retrieved on 2018-01-19]
- DATABASE MEDLINE [Online] US NATIONAL LIBRARY OF MEDICINE (NLM), BETHESDA, MD, US; June 2001 (2001-06), SEIDMAN S N ET AL: "Testosterone replacement therapy for hypogonadal men with major depressive disorder: a randomized, placebo-controlled clinical trial.", Database accession no. NLM11465516 & STUART N. SEIDMAN ET AL: "Testosterone Replacement Therapy for Hypogonadal Men With Major Depressive Disorder : A Randomized, Placebo-Controlled Clinical Trial", JOURNAL OF CLINICAL PSYCHIATRY, vol. 62, no. 6, 15 June 2001 (2001-06-15), pages 406-412, XP055551219, US ISSN: 0160-6689, DOI: 10.4088/JCP.v62n0602

## Description

### FIELD OF THE INVENTION

The present invention is related to compositions for use in a method of treating a depressive symptom in a subject by administering to the subject an effective amount of a steroid in the testosterone synthetic pathway.

### DESCRIPTION OF THE RELATED ART

In the 1940's several studies demonstrated that testosterone and other androgens may be successfully used to treat depressive syndromes in middle-aged men. But with increasing use of electroconvulsive therapy and the advent of tricyclic antidepressants and monoamine oxidase inhibitors in the 1950's, androgens lost favor as a treatment for depression. A few studies in the 1970's and 80's reconfirmed the efficacy of androgens such as mesterolone in depressed men, but androgens continued to arouse little interest, perhaps because of the steady introduction of newer classes of antidepressant agents, of which some could be administered to both sexes without concern for masculinizing effects.

In other studies some depressed men exhibited reduced testosterone levels, although this association is complex and probably affected by additional factors. Hypogonadal men also often exhibit depressive symptoms and testosterone replacement therapy generally improves these symptoms. This finding extends to men with HIV-induced hypogonadism, who also appear to show an antidepressant response to testosterone. Furthermore, men who ingest markedly supraphysio logic doses of testosterone and related androgens (such as illicit anabolic steroid abusers) may develop manic or hypomanic symptoms during androgen use and depressive symptoms on androgen withdrawal.

In more recent studies, the potential of testosterone as an antidepressant has been reconsidered. In one study by Seidman, et al. (Seidman SN, Rabkin J., J Affective Disord 1998;48:157-161), intramuscular testosterone enanthate, was administered at 400 mg every two weeks to five men who had remained depressed despite adequate treatment with selective serotonin reuptake inhibitors (SSRI's). These men's total testosterone levels were in the low or borderline range (200-350 ng/dl; reference range 300-990 ng/dl). All five subjects improved. Their mean depression scores on the Hamilton Rating Scale for Depression (HAM-D) declined from 19.2 at baseline to 4.0 at eight weeks. Subsequently, four of the five men were administered placebo injections, and three of these four relapsed within two weeks. Following this study Seidman, et al. (J Clin Psychiatry 2001;62:406-412), conducted a randomized, placebo-controlled trial of testosterone enanthate in men with major depressive disorder, again selecting subjects with testosterone levels of 350 ng/dl or less. However, this study differed from the prior open-label study in that subjects were not simultaneously taking an antidepressant medication, but received testosterone alone. After six weeks of treatment, the investigators found no significant difference between testosterone and placebo on the Hamilton Rating Scale for Depression or Beck Depression Inventory (BDI). About 40% of testosterone-treated subjects responded (as defined by a 50% or greater reduction in the Hamilton Rating Scale for Depression), but so did a comparable portion of subjects receiving placebo. Interestingly, of eight placebo non-responders offered open-label testosterone at the conclusion of the study, six responded. While admitting that these latter observations were subject to expectational bias, the authors speculated that testosterone possessed variable and possibly idiosyncratic antidepressant effects in some men, and that further research was justified.

Transdermal preparations of testosterone have provided a useful delivery system for normalizing serum testosterone levels in hypogonadal men and preventing the clinical symptoms and long term effects of androgen deficient men. Available transdermal preparations of testosterone include, for example, TESTODERM®, TESTODERM® TTS, and ANDRODERM®. Testosterone is also available in other formulations including those available as an injectable, for example, DEPO-TESTOSTERONE® (testosterone cypionate), and DELATESTRYL BTG® (testosterone enanthate), or as a gel, for example, ANDROGEL® marketed by Unimed Pharmaceuticals, Inc., Deerfield, Illinois, the assignee of this application. WO 02/17926 describes ANDROGEL® for use in treating hypogonadism.

In men, transdermal patches are applied to the scrotal skin or other parts of the body. Recently, a one-percent testosterone gel has been approved for use in men, and provides dosing flexibility with minimal skin irritation. This gel is marketed under the name ANDROGEL®. However, all currently available testosterone transdermal products are specifically contraindicated for use in women in the United States. Furthermore, none of the currently available androgen treatment modalities for women, for example, oral methyltestosterone, intramuscular testosterone ester injections or subcutaneous testosterone implants can achieve reproducible testosterone serum levels on a consistent daily basis.

Testosterone, the major circulating androgen in men, is synthesized from cholesterol. The approximately 500 million Leydig cells in the testes secrete more than 95% of the 6-7 mg of testosterone produced per day. Two hormones produced by the pituitary gland, luteinizing hormone ("LH") and follicle stimulating hormone ("FSH"), are required for the development and maintenance of testicular function and negatively regulate testosterone production. Circulating testosterone is metabolized to various 17-keto steroids through two different pathways. Testosterone can be metabolized to dihydrotestosterone ("DHT") by the enzyme 5-alpha-reductase or to estradiol ("E₂") by an aromatase enzyme complex.

Testosterone circulates in the blood 98% bound to protein. In men, approximately 40% of the binding is to the high-affinity sex hormone binding globulin ("SHBG"). The remaining 60% is bound weakly to albumin. Thus, a number of measurements for testosterone are available from clinical laboratories. The term "free" testosterone as used herein refers to the fraction of testosterone in the blood that is not bound to protein. The term "total testosterone" or "testosterone" as used herein means the free testosterone plus protein-bound testosterone. The term "bioavailable testosterone" as used herein refers to the non-sex hormone binding globulin bound testosterone and includes testosterone weakly bound to albumin.

The following table from the UCLA-Harbor Medical Center summarizes the hormone concentrations in normal adult men range:

**Table 1: Hormone Levels in Normal Men**

| **Hormone** | **Normal Range** |
|---|---|
| Testosterone | 298 to 1043 ng/dL |
| Free testosterone | 3.5 to 17.9 ng/dL |
| DHT | 31 to 193 ng/dL |
| DHT/T Ratio | 0.052 to 0.33 |
| DHT + T | 372 to 1349 ng/dL |
| SHBG | 10.8 to 46.6 nmol/L |
| FSH | 1.0 to 6.9 mlU/mL |
| LH | 1.0 to 8.1 mlU/mL |
| E₂ | 17.1 to 46.1 pg/mL |

There is considerable variation in the half-life of testosterone reported in the literature, ranging from 10 to 100 minutes. Researchers do agree, however, that circulating testosterone has a diurnal variation in normal young men. Maximum levels occur at approximately 6:00 to 8:00 a.m. with levels declining throughout the day. Characteristic profiles have a maximum testosterone level of 720 ng/dL and a minimum level of 430 ng/dL. The physiological significance of this diurnal cycle, if any, however, is not clear.

Because increasing testosterone concentrations has been shown to alter sexual performance and libido, researchers have investigated methods of delivering testosterone to men. These methods include intramuscular injections (43%), oral replacement (24%), pellet implants (23%), and transdermal patches (10%). A summary of these methods is shown in Table 2.

**Table 2: Mode of Application and Dosage of Various Testosterone Preparations**

| **Preparation** | **Route Of Application** | **Full Substitution Dose** |
|---|---|---|
| **In Clinical Use** | | |
| Testosterone enanthate | Intramuscular injection | 200-25.0 g every 2-3 weeks |
| Testosterone cypionate | Intramuscular injection | 200 mg every 2 weeks |
| Testosterone undecanoate | Oral | 2-4 capsules at 40 mg per day |
| Transdermal testosterone patch | Scrotal skin | 1 membrane per day |
| Transdermal testosterone patch | Non-scrotal skin | 1 or 2 systems per day |
| Testosterone implants | Implantation under the abdominal skin | 3-6 implants of 200 mg every 6 months |

| **Under Development** | | |
|---|---|---|
| Testosterone cyclodextrin | Sublingual | 2.5-5.0 mg twice daily |
| Testosterone undecanoate | Intramuscular injection | 1000 mg every 8-10 weeks |
| Testosterone buciclate | Intramuscular injection | 1000 mg every 12-16 weeks |
| Testosterone microspheres | Intramuscular injection | 315 mg for 11 weeks |

| **Obsolete** | | |
|---|---|---|
| 17□-Methyltestosterone | Oral | 25-5.0 g per day |
| Fluoxymesterone | Sublingual | 10-25 mg per day |
| | Oral | 10-20 mg per day |

All of the testosterone replacement methods currently employed, however, suffer from one or more drawbacks. For example, subdermal pellet implants and ester injections are painful and require doctor visits. Many of these methods, such as oral/sublingual/buccal preparations, suffer from undesirable pharmacokinetic profile-creating supra-physiologic testosterone concentrations followed a return to baseline. Transdermal patches provide less than optimal pharmacokinetic characteristics, are embarrassing for many subjects, and are associated with significant skin irritation. Thus, although the need for an effective testosterone replacement methodology has existed for decades, an alternative replacement therapy that overcomes these problems has never been developed.

For these and other reasons, therefore, it would be a difficult but much desired advance in the art to provide an effective percutaneously administered steroid in the testosterone synthetic pathway formulation to be applied directly to the skin of a subject in the form of, for example, a gel, an ointment, or a cream, to treat a depressive symptom, and in particular to treat a subject that has failed to respond to conventional antidepressants and/or who exhibited low or borderline testosterone levels.

### BRIEF DESCRIPTION OF THE FIGURES

Figure No. 1 is a flow diagram showing subject progress through an eight-week randomized placebo-controlled depression trial of testosterone transdermal gel.
Figure No. 2 is a line graph showing the Hamilton Depression Rating Scale scores in an eight-week randomized placebo-controlled depression trial of testosterone transdermal gel.
Figure No. 3 is a line graph showing the Clinical Impression scores in eight-week randomized placebo-controlled depression trial of testosterone transdermal gel.
Figure No. 4 is a line graph showing the Beck Depression Inventory scores an eight-week randomized placebo-controlled depression trial of testosterone transdermal gel.

### DETAILED DESCRIPTION OF THE PRESENT INVENTION

The present invention is directed to compositions for use in a method of treating, a depressive disorder as defined in the claims. The method comprises administering, for example, percutaneously, to a subject a depressive-disorder-effective amount of testosterone. The present invention includes methods of reversing, halting or slowing the progression of a depressive disorder once it becomes clinically evident, or treating the symptoms associated with, or related to the depressive disorder. In accordance with the invention, the method comprises administering to the subject a depressive-disorder-effective amount of a percutaneously deliverable composition comprised of testosterone, one or more lower alcohols, such as ethanol or isopropanol, a penetration enhancing agent, a thickening agent, and water, as defined in the claims. In one embodiment the testosterone composition is formulated as a gel, ointment, cream, or patch. In yet another embodiment the testosterone composition is a hydroalcoholic gel. In another embodiment, the composition is a gel comprising testosterone, one or more lower alcohols, such as ethanol or isopropanol, a penetration enhancing agent, a thickening agent, and water. accordance with the invention, the compositions are used in conjunction with an antidepressant agent.

In one embodiment, the composition of the present invention is administered once, twice, or three times a day, or as many times necessary to achieve the desired therapeutic effect. In another embodiment the composition of the present invention is administered once, twice, or three times a day on alternate days. In another embodiment the composition of the present invention is administered once, twice, or three times a day on a weekly, biweekly, or monthly basis.

In one embodiment, the present invention employs testosterone in conjunction with a pharmacologically-effective amount of antidepressant agent in the same dosage form or in a separate dosage form.

In another embodiment, the compositions are used with another steroid or pharmaceutical agent that increases testosterone levels in a subject, for example, an agent that inhibits the synthesis of sex hormone binding globulin, for example, methyltestosterone or fluoxymesterone.

In yet another embodiment, the present invention employs a packet having a polyethylene liner compatible with the components of the gel. In another embodiment, the compositions employ a composition that is dispensed from a rigid multi-dose container (for example, with a hand pump) having a larger foil packet of the composition inside the container. Such larger packets can also comprise a polyethylene liner as above.

Additionally, the compositions may optionally include a salt, an ester, an amide, an enantiomer, an isomer, a tautomer, a prodrug, or a derivative of an agent of the present invention, as well as an emollient, a stabilizer, an antimicrobial, a fragrance, or a propellant.

The methods, and compositions of the present invention provide enhanced treatment options for treating a depressive disorder in a man as compared to those currently available.

Antidepressant agents useful in the methods, of the present invention include, for example, bicyclics, such as binedaline, caroxazone, citalopram, dimethazan, fencamine, indalpine, indeloxzine hydrochloride, nefopam, nomifensine, oxitriptan, oxypertine, paroxetine, sertraline, thiazesim, and trazodone; hydrazides/hydrazines, such as benmoxine, iproclozide, iproniazid, isocarboxazid, nialamide, octamoxin, and phenelzine; pyrrolidones, such as cotinine, rolicyprine, or rolipram; tetracyclics, such as maprotiline, metralindole, mianserin, and mitrazepine; tricyclics, such as adinazolam, amitriptyline, amitriptylinoxide, amoxaprine, butriptyline, clomipramine, demexiptiline, desipramine, dibenzepin, dimetacrine, dothiepin, doxepin, fluacizine, imipramine, imipramine N-oxide, iprindole, lofepramine, melitracen, metapramine, nortriptyline, noxiptilin, opipramol, pizotyline, propizepine, protriptyline, quinupramine, tianeptine, and trimipramine; and others, such as adrafinil, amoxapine, benactyzine, bupropion, butacetin, dioxadrol, duloxetine, etoperidone, febarbamate, femoxetine, fenpentadiol, fluoxetine, fluvoxamine, hematoporphyrin, hypericin, levophacetoperane, medifoxamine, milnacipran, minaprine, moclobemide, maprotline, mirtazapine, nefazodone, oxaflozane, phenelzine, piberaline, prolintane, pyrisuccideanol, ritanserin, roxindole, rubidium chloride, sulpride, tandospirone, thozalinone, tofenacin, toloxatone, tranylcypromine, trazodone, L-tryptophan, venlafaxine, viloxazine, and zimeldine; and all salts, esters, amides, enantiomers, isomers, tautomers, prodrugs and derivatives of these compounds. (Based in part upon the list provided in The Merck Index, Merck & Co. Rahway, N.J. (1998)). Combinations of the above mentioned antidepressant agents can be used in the methods, kits, combinations, and compositions herein described.

Other classes of antidepressant agents useful in the methods of the present invention include, for example, anitparkinsonian agents, such as amantadine, benserazide, bietanautine, biperiden, bromocriptine, budipine, carbidopa, dexetimide, diethazine, droxidopa, ethopropazine, ethylbenzhydramine, lazabemide, levodopa, mofegiline, pergolide, piroheptine, pramipexole, pridinol, prodipine, ropinirole, selegiline, talipexole, terguride, and trihexyphenidyl hydrochloride; antipsychotic agents such as benzamides: alizapride, amisulpride, nemoapride, remoxipride, sulpiride, and sultopride; benzisoxazoles, such as risperidone; butyrophenones, such as benperidol, bromperidol, droperidol, fluanisone, haloperidol, melperone, moperone, pipamperone, spiperone, timiperone, and trifluperidol; phenothiazines, such as acetophenazine, butaperazine, carphenazine, chlorproethazine, chlorpromazine, clospirazine, cyamemazine, dixyranzine, fluphenazine, imiclopazine, mepazine, mesoridazine, methoxypromazine, metofenazate, oxaflumazine, perazine, pericyazine, perimethazine, perphenazine, piperacetazine, pipotiazine, prochlorperazine, promazine, sulforidazine, thiopropazate, thioproperazine, thioridazine, trifluoperazine, and triflupromazine; thioxanthenes, such as chlorprothixene, clopenthixol, flupentixol, thiothixene; other tricyclics, such as benzquinamide, carpipramine, clocapramine, clomacran, clothiapine, clozapine, mosapramine, olanzapine, opipramol, prothipendyl, seroquel®, tetrabenazine, and zotepine; and other antiparkinsonian agents, such as buramate, fluspirilene, molindone, penfluridol, pimozide, ziprasidone; dopamine receptor angonists, such as bromocriptine, cabergoline, carmoxirole, dopexamine, fenoldopam, ibopamine, lisuride, pergolide, pramipexole, quinagolide, ropinrole, roxindole, and talipexole; dopamine receptor antagonist, such as amisulpride, clebopride, domperidone, metoclopramide, mosapramine, nemonapride, remoxipride, risperidone, sulpiride, sultopride, and ziprasidone; monoamine oxidase inhibiting agents, such as iproclozide, iproniazid, isocarboxazid, lazabemide, mofegiline, moclobemide, octamoxin, pargyline, phenelzine, phenoxypropazine, pivalylbenzhydrazine, prodipine, selegiline, and toloxatone, tranylcypromine; and selective serotonin reuptake inhibitors, such as, citalopram, fluoxetine, fluvoxamine, venlafaxine, sertraline, paroxetine; and all salts, esters, amides, enantiomers, isomers, tautomers, prodrugs and derivatives of these compounds. (Based in part upon the list provided in The Merck Index, Merck & Co. Rahway, N.J. (1998)). Combinations of the above mentioned antidepressant agents can be used in the methods herein described.

Illustratively, antidepressant agents of particular interest that can be used in the methods of the present invention include, but are not limited to Ativan®, Librium®, Limbitrol®, Tranxene®, Valium®, Xanax®, Atarax®, BuSpar®, Effexor®, Mebaral®, Miltown®, Paxil®, Sinequan®, Triavil®, Vistaril®, Remeron®, Serzone®, Wellbutrin®, Nardil®, Parnate®, Celexa®, Prozac®, Zoloft®, Elavil®, Etrafon®, Norpramin®, Surmontil®, Vivactil®, Depakote®, Eskalith0, lithium, Lithobid®, Klonopin®, Clozaril®, Haldol®, Loxitane®, Moban®, Navane®, Orap®, Risperdal®, Seroquel®, Zyprexa®, Compazine®, Serentil®, Stelazin®, Thioridazine®, Trilafon®, and Luvox®. Combinations of the above mentioned antidepressant agents can be used in the methods herein described.

A class of steroids or pharmaceutical agents that increases testosterone levels in a subject useful in the methods and compositions of the present invention include compounds that inhibit the synthesis of the sex hormone binding globulin. Sex hormone binding globulin is a serum protein, and is known to bind to testosterone and estradiol, effecting the biological activity of these hormones. Specific compounds of interest that inhibit the synthesis the sex hormone binding globulin include but are not limited to methyltestosterone and fluoxymesterone, and all salts, esters, amides, enantiomers, isomers, tautomers, prodrugs and derivatives of these compounds. Combinations of the above compounds can be used in the methods and compositions herein described. Methyltestosterone is currently available in various formulations including those available orally, for example, ANDROID® and TESTRED®. Fluoxymesterone is also currently available in various formulations including those available orally, for example, HALOSTESTIN®.

While not wishing to be bound by theory, it is believed that methyltestosterone decreases hepatic synthesis of endogenous proteins like sex hormone binding globulin. This decrease in synthesis produces a decline in blood concentrations of sex hormone binding globulin, which is the primary means of endogenous hormone transport. The decrease in sex hormone binding globulin subsequently causes an increase in free-hormone concentration for binding at the receptor. Transdermal application of an androgen, for example, testosterone, or an estrogen, for example, estradiol, bypasses first-pass metabolism and can provide a means of increasing hormone concentrations in the bloodstream. Thus, when used in combination, methyltestosterone and percutaneously administered testosterone (and optionally estradiol) produce a greater therapeutic effect and provide a means of increasing hormone concentrations in the bloodstream. Methyltestosterone and testosterone (and optionally estradiol) produce a greater therapeutic effect than either entity alone because the decrease in hormone binding ability is coupled with an increased hormone bioavailability, producing higher free-hormone concentrations that would be produced by testosterone alone.

In another embodiment of the present invention, the estrogenic hormone that can be used in conjunction with the methods, and composition is the naturally occurring estrogen 17 beta-estradiol (beta-estradiol; 1, 3, 5(10)-estratriene-3, 17 beta-diol). Other estrogenic steroid hormones can be used in partial or complete replacement of 17 beta-estradiol, for example, an ester which is biologically compatible and can be absorbed effectively transdermally. The estradiol esters can be, illustratively estradiol-3,17-diacetate; estradiol-3-acetate; estradiol-17-acetate; estradio1-3,1 7-divalerate; estradiol-3-valerate; estradiol-17-valerate; 3-mono, 17-mono and 3,17-dipropionate esters, corresponding cypionate, heptanoate, benzoate and the like esters; ethynil estradiol; estrone and other estrogenic steroids and salts, enantiomers, isomers, tautomers, prodrugs and derivatives thereof that are possible to administer by transdermal route. Other estrogen-related compounds that may be used in the methods and compositions of the present invention include, but are not limited to conjugated estrogens (including estrone sulfate, equilin, and 17-.alpha.-dihydroequilin), estradiol valerate, estriol, estrone, estrone sulfate, estropipate, ethinyl estradiol, mestranol, and all salts, esters, amides, enantiomers, isomers, tautomers, prodrugs and derivatives of these compounds.

Estrogenic hormones are currently available in various formulations including, but not limited to those available as a cream, pessary, vaginal ring, vaginal tablet, transdermal preparation, gel, and oral tablet. Examples of vaginal creams include PREMARIN® (conjugated estrogen), ORTHO DIENOSTEROL® (dienosterol), and OVESTIN® (estriol). Available pessary formulations include ORTHO-GYNEST® (estriol), and TAMPOVAGAN® (stilbestrol). An example of a vaginal ring formulation is ESTRING® (estradiol), and an example of a vaginal tablet is VAGIFEM® (estradiol). Available transdermal estrogen preparations containing estradiol include ERC ALORA®, CLIMARA®, DERMESTRIL®, ESTRADERM®, ESTRADERM® TTS, ESTRADERM® MX, EVOREL®, FEMATRIX®, FEMPATCH®, FEVISEVEN®, MENOREST®, PROGYNOVA® TS, and VIVELLE®. Estrogen gels containing estradiol include ESTRAGEL (under development by Applicant), and SANDRENA®. Estradiol is also available formulated as an implant pellet, for example, ESTRADIOL IMPLANT®. Tablet formulations include PREMARIN® (conjugated estrogen), ESTRATAB® (esterified estrogen), ESTRATEST® (esterified estrogen, methyltestosterone), MENEST® (esterified estrogen), CLIMAGEST®, (estradiol), CLIMAVAL® (estradiol), ELLESTE SOLO® (estradiol), ESTRACE® (estradiol), PROGYNOVA® (estradiol), ZUMENON® (estradiol), HORMONIN® (estradiol, estrone, estriol), HARMOEN® (estrone), OGEN® (estropipate), and ORTHO-EST® (estropipate).

Combinations of the above mentioned estrogenic hormones can be used in the methods and compositions herein described.

In one embodiment, testosterone is formulated as a hydroalcoholic gel. In another embodiment, the gel comprises testosterone, one or more lower alcohols, such as ethanol or isopropanol, a penetration enhancing agent, a thickening agent, and water. Additionally, the gel optionally includes the a salt, an ester, an amide, an enantiomer, an isomer, a tautomer, a prodrug, or a derivative of testosterone, as well as an emollient, a stabilizer, an antimicrobial, a fragrance, or a propellant.

Illustratively, certain formulations of the present invention deliver about 0.01 g to about 100 g testosterone to a subject per dosage unit. In another embodiment of the present invention, the formulations deliver from about 0.1 g to about 10 g testosterone to a subject per dosage unit. In yet another embodiment of the present invention, the formulations of the present invention deliver from about 0.17 g to about 5 g testosterone to a subject per dosage unit. In another embodiment of the present invention, the formulations of the present invention deliver about 1 g testosterone to a subject per dosage unit. In still another embodiment of the present invention, the formulations of the present invention deliver about 0.25 g testosterone to a subject per dosage unit. Thus, for example, a testosterone gel, ointment, cream or patch is formulated as a single dosage unit for once a day administration contains about 0.17 g, or about 0.25 g, or about 0.5 g testosterone, or about 1.0 g testosterone, while a gel, ointment, cream or patch formulated as a single dosage unit for once a week administration contains about 1.19 g, or about 1.75 g, or about 3.50 g, or about 7.0 g testosterone, respectfully.

In one embodiment, the formulation is a gel, an ointment, a cream or a patch and is comprised of testosterone; a penetration enhancing agent, such as isopropyl myristate; a thickening agent, such as Carbopol; a lower alcohol, such as ethanol or isopropanol; and water. In another embodiment the formulation is a gel, an ointment, a cream or a patch and is comprised of the following substances in approximate percentages:

**Table 3 : Composition of Testosterone Formulation**

| **SUBSTANCE** | **AMOUNT (w/w)** |
|---|---|
| Testosterone | 0.01 - 70% |
| Penetration enhancing agent | 0.01 - 50% |
| Thickening agent | 0.01 - 50% |
| Lower alcohol | 30 - 98% |
| Purified water (qsf) | 100% |

Illustratively, in a 100 g composition, the gel, ointment, cream, or patch may contain about 0.01 g to about 70 g of testosterone, about 0.01 g to about 50 g penetration enhancing agent, about 0.1 g to about 50 g thickening agent, and about 30 g to about 98 g lower alcohol. In another embodiment, in a 100 g composition, the gel, ointment, cream, or patch may contain about 0.1 g to 10 g of testosterone, about 0.1 g to about 5 g of penetration enhancing agent, about 0.1 g to about 5 g of thickening agent, an about 45 g to about 90 g lower alcohol.

In yet another embodiment, the composition is a gel, ointment, cream, or patch that further comprises a hydroxide releasing agent, such as sodium hydroxide (fore example, 0.1 N NaOH), in an amount of about 0.1% to about 10% w/w of the composition.

In one embodiment, the formulation is a gel and is comprised of the following substances in approximate weights:

**Table 4: Composition of AndroGel®**

| **SUBSTANCE** | **AMOUNT (w/w) PER 100g OF GEL** |
|---|---|
| Testosterone | 1.0 g |
| Isopropyl myristate | 0.50 g |
| Carbopol 980 | 0.90 g |
| 0.1 N NaOH | 4.72 g |
| Ethanol (95% w/w) | 72.5 g* |
| Purified water | q.s. |

| | |
|---|---|
| *Corresponding to 67 g of ethanol. | |

In another embodiment, the formulation is a gel and is comprised of the following substances in approximate weights:

**Table 5 : Composition of Relibra®**

| **SUBSTANCE** | **AMOUNT (w/w) PER 100g OF GEL** |
|---|---|
| Testosterone | 0.1 g |
| Isopropyl myristate | 0.50 g |
| Carbopol 980 | 0.90 g |
| 0.1 NNaOH | 4.72 g |
| Ethanol (95% w/w) | 72.5 g* |
| Purified water | q.s. |

| | |
|---|---|
| *Corresponding to 67 g of ethanol. | |

In still another embodiment, the composition comprises testosterone in an amount greater than 0.01%, a penetration enhancing agent in an amount greater than about 0.1%, a thickening agent in an amount greater than about 0.1%, and a lower alcohol in an amount greater than about 30% w/w of the composition.

The gel, ointment, cream, or patch is rubbed or placed onto an area of skin of the subject and allowed to dry. Illustratively, the gel, ointment, or cream is rubbed onto an area of skin, for example, on the upper outer thigh and/or hip once daily. Following application the subject washes his or her hands. Application of the gel results in an increased testosterone level having a desirable pharmacokinetic profile effective to treat a depressive disorder, or the symptoms associated with, or related to a depressive disorder in the subject.

In one embodiment of the present invention a composition is provided for use in a method for treating a depressive disorder as defined in the claims. The method comprises administering a depressive-disorder-effective amount of a composition to an area of skin of the subject for delivery of testosterone to blood serum of the subject. The composition comprises:
(a) about 0.01% to about 70% (w/w) testosterone;
(b) about 0.01% to about 50% (w/w) penetration enhancing agent;
(c) about 0.01% to about 50% (w/w) thickening agent; and
(d) about 30% to about 98% (w/w) lower alcohol.
The composition is capable of releasing the steroid after applying the composition to the skin at a rate and duration that delivers at least about 10 µg per day of testosterone to the blood serum of the subject.

In another embodiment of the present invention, the composition is capable of releasing the testosterone after applying the composition to the skin of a subject at a rate and duration that achieves a circulating serum concentration of testosterone greater than about 400 ng per dl serum during a time period beginning about 2 hours after administration and ending about 24 hours after administration.

In another embodiment of the present invention, the composition is capable of releasing the testosterone after applying the composition to the skin of a subject at a rate and duration that achieves a circulating serum concentration of the testosterone between about 400 ng testosterone per dl serum to about 1050 ng testosterone per dl serum.

In another embodiment of the present invention, for each about 0.1 gram per day application of the composition of the present invention to the skin of a subject, an increase of at least about 5 ng/dl in serum testosterone concentration results in the subject.

In another embodiment of the present invention, the composition of the present invention is provided to a subject for daily administration in about a 0.1 g to about a 10 g dose.

In yet another embodiment of the present invention, the subject in need of treatment has a serum testosterone level before the first application (pretreatment) of the composition of the present invention of less than about 300 ng/dl.

In another embodiment of the present invention, after at least about 30 days of daily administration of the composition of the present invention the serum testosterone concentration in a subject is at least about 490 ng/dl to about 860 ng/dl.

In still another embodiment of the present invention, after at least about 30 days of daily administration of the composition of the present invention the total serum androgen concentration in a subject is greater than about 372 ng/dl.

In another embodiment of the present invention, the composition of the present invention is administered once, twice, or three times daily to a subject for at least about 7 days.

The present invention also provides a composition for use in a method of treating a depressive disorder in a subject in need thereof as defined in the claims, by administering to the subject:
(a) an amount of a composition comprising:
   (i) about 0.01% to about 70% (w/w) testosterone;
   (ii) about 0.01% to about 50% (w/w) penetration enhancing agent;
   (iii) about 0.01% to about 50% (w/w) thickening agent; and
   (iv) about 30% to about 98% (w/w) lower alcohol; and
(b) an amount of a therapeutic agent comprising an antidepressant and, optionally, an inhibitor of the synthesis of sex hormone binding globulin, or an estrogenic hormone.

The composition is administered to an area of skin of the subject for delivery of testosterone to the blood serum of the subject, and is capable of releasing testosterone after applying the composition to the skin at a rate and duration that delivers at least about 10 µg per day of the steroid to the blood serum of the subject. The amount of the composition and the amount of the therapeutic agent together make a depressive-disorder-effective amount.

In one embodiment of the present invention, the composition and the therapeutic agent are provided as separate components to a kit.

In another embodiment of the present invention, the composition and the therapeutic agent are administered substantially simultaneously, or sequentially.

In still another embodiment of the present invention, the therapeutic agent is administered orally, percutaneously, intravenously, intramuscularly, or by direct absorption through mucous membrane tissue.

The present invention also provides a pharmaceutical composition for use as defined in the claims, comprising:
(i) about 0.01% to about 70% (w/w) testosterone;
(ii) about 0.01 % to about 50% (w/w) penetration enhancing agent;
(iii) about 0.01% to about 50% (w/w) thickening agent;
(iv) about 30% to about 98% (w/w) lower alcohol; and
(v) a therapeutic agent comprising an antidepressant, and optionally an inhibitor of the synthesis of sex hormone binding globulin, or an estrogenic hormone.

The composition is administered to an area of skin of the subject for delivery of the testosterone and the therapeutic agent to the blood serum of the subject, and is capable of releasing the steroid after applying the composition to the skin at a rate and duration that delivers at least about 10 µg per day of the steroid to the blood serum of the subject. The amount of the testosterone and the amount of the therapeutic agent together make a depressive-disorder-effective amount.

Achieving target delivery rates demonstrated by testosterone gel can be estimated from the pharmacokinetics in testosterone gel in men. The mean serum concentration (Cavg) values in men after applying of varying amounts of gel to the upper body is given below in Table 6.

**Table 6**

| **Mean Averag**e **Serum Testosterone Concentrations and Daily Delivery Rate after Administration of Testosterone Gel 1% in Men** | | |
|---|---|---|
| Dose (µL) (gram) | Mean Cavg (ng/dL) | Daily Delivery Rate (µg/day)^{a} |
| 5.0 | 555 (± 225) | 3330 |
| 7.5 | 601 (± 309) | 3606 |
| 10 | 713 (± 209) | 4278 |

| | | |
|---|---|---|
| ^{a} Metabolic Clearance Rate of Daily Testosterone = 600 L/day | | |

Based on the results obtained in men, a testosterone gel dose of 0.5 grams delivers approximately 300 µg of testosterone per day.

Thus, to achieve a serum blood level of about 100 µg testosterone, the composition is administered at about 0.17 g/day, which delivers about 1.7 mg/day of testosterone to the skin of which about 0.1 mg, is absorbed; or to achieve a serum blood level of about 150 µg testosterone, the composition is administered at about 0.25 g/day, which delivers about 2.5 mg/day of testosterone to the skin of which about 0.15 mg, is absorbed; or to achieve a serum blood level of about 300 µg testosterone, the composition is administered at about 0.5 g/day, which delivers 5.0 mg/day of testosterone to the skin of which about 0.3 mg, is absorbed.

The phrase "depressive disorder" refers to a condition, disorder, or disease such as a mood disorder, decreased libido, melancholia, reactive depression, endogenous depression, endogenomorphic depression, anaclitic depression, or any depressive symptom sufficient to meet one or more of the DSM-IV criteria for current major depressive disorder, or any depressive symptom that increases a depression score on the Hamilton Rating Scale or the Depression Beck Depression Inventory.

The term "treat" or "treatment" as used herein refers to any treatment of a mammalian condition, disorder, or disease associated with a depressive disorder, and includes, but is not limited to, inhibiting the condition, disorder, or disease, for example, arresting the development of the condition, disorder, or disease; relieving the condition, disorder, or disease, for example, causing regression of the condition, disorder, or disease; or relieving the condition caused by the disease or disorder, for example, stopping the symptoms of the disease or disorder. In one embodiment "treat" or "treatment" includes, for example, improving or alleviating a mood disorder, increasing libido, improving or alleviating one or more symptoms of melancholia, improving or alleviating one or more symptoms of reactive depression, improving or alleviating one or more symptoms of endogenous depression, improving or alleviating one or more symptoms of endogenomorphic depression, improving or alleviating one or more symptoms of anaclitic depression, or improving or alleviating any depressive symptom that meets the DSM-IV criteria for current major depressive disorder, or improving or alleviating any depressive symptom that increases a depression score on the Hamilton Rating Scale or the Depression Beck Depression Inventory.

A "depressive-disorder effect" or "depressive-disorder-effective amount" is intended to qualify the amount of an agent required to treat a depressive disorder in a subject, or relieve to some extent one or more of the symptoms associated with, or related to, a depressive disorder in a subject. In a mammal, this includes, but is not limited to, improving or alleviating a mood disorder, increasing libido, improving or alleviating one or more symptoms of melancholia, improving or alleviating one or more symptoms of reactive depression, improving or alleviating one or more symptoms of endogenous depression, improving or alleviating one or more symptoms of endogenomorphic depression, improving or alleviating one or more symptoms of anaclitic depression, or improving or alleviating any depressive symptom that meets the DSM-IV criteria for current major depressive disorder, or improving or alleviating any depressive symptom that increases a depression score on the Hamilton Rating Scale or the Depression Beck Depression Inventory. Treatment of a subject with the methods, and compositions of the present invention also include, for example, normalizing hypogonadism; improving sexual dysfunction; normalizing cholesterol levels; normalizing abnormal electrocardiograms of subjects and improving vasomotor symptoms; improving diabetic retinopathy as well as lowering the insulin requirements of diabetic subjects; decreasing the percentage of body fat; normalizing glucose levels; decreasing the risk factors for cardiovascular disease, including normalizing hypertension, and treating obesity; preventing osteoporosis, osteopenia, vaginal dryness, and thinning of the vaginal wall; relieving menopausal symptoms and hot flashes; improving cognitive dysfunction; treating, preventing or reducing the onset of cardiovascular disease, Alzheimer's disease, dementia, and cataracts; and treating, preventing or reducing the risk of cervical, uterine or breast cancer.

When the compositions of the present invention are used in a "depressive-disorder effective amount" this means that the concentration of the therapeutic agent is such that a therapeutic level of agent is delivered over the term that the composition is to be used. Such delivery is dependent on a number of variables including the time period for which the individual dosage unit is to be used, the flux rate of the therapeutic agent, for example, testosterone, from the gel, surface area of application site, etc. The amount of therapeutic agent necessary can be experimentally determined based on the flux rate of the drug through the gel, for example, and through the skin when used with and without enhancers. It is understood, however, that specific dose levels of the therapeutic agents of the present invention for any particular subject depends upon a variety of factors including the activity of the specific compound employed, the age, body weight, general health, sex, and diet of the subject, the time of administration, the rate of excretion, the drug combination, and the severity of the particular disorder being treated and form of administration. Treatment dosages generally may be titrated to optimize safety and efficacy. Typically, dosage-effect relationships from *in vitro* and/or *in vivo* tests initially can provide useful guidance on the proper doses for subject administration. In terms of treatment protocols, it should be appreciated that the dosage to be administered will depend on several factors, including the particular agent that is administered, the route administered the condition of the particular subject, etc. Generally speaking, one will desire to administer an amount of the agent that is effective to achieve a serum level commensurate with the concentrations found to be effective *in vitro.* Thus, where an agent is found to demonstrate *in vitro* activity at, for example, 10 ng/ml, one will desire to administer an amount of the agent that is effective to provide about a 10 ng/ml concentration *in vivo.* Determination of these parameters is well within the skill of the art. These considerations, as well as effective formulations and administration procedures are well known in the art and are described in standard textbooks.

In order to measure and determine the amount of testosterone to be delivered to a subject to administer a depressive-disorder effective amount to the subject, serum testosterone concentrations can be measured using standard assay techniques. For example, free serum testosterone levels are measured by the recently validated and highly sensitive equilibrium dialysis method discussed in Sinha-Hikim et al., The Use of a Sensitive Equilibrium Dialysis Method for the Measurement of Free Testosterone Levels in Healthy, Cycling Women and in HIV-Infected Women, 83 J. CLINICAL ENDOCRINOLOGY & METABOLISM 1312-18. (1998).

As used herein, the phrases "androgen deficiency" or "testosterone deficiency" are used interchangeably, and refer to lower serum levels of free testosterone in a subject as compared to the median serum levels for healthy subject of the same age. Diagnosis of a testosterone deficiency is known to the average physician practicing in the relevant field of medicine.

The use of the term "about" in the present disclosure means "approximately," and use of the term "about" indicates that dosages slightly outside the cited ranges may also be effective and safe, and such dosages are also encompassed by the scope of the present claims.

The term "prodrug" refers to a drug or compound in which the pharmacological action (active curative agent) results from conversion by metabolic processes within the body. Prodrugs are generally considered drug precursors that, following administration to a subject and subsequent absorption, are converted to an active or a more active species via some process, such as a metabolic process. Other products from the conversion process are easily disposed of by the body. Prodrugs generally have a chemical group present on the prodrug which renders it less active and/or confers solubility or some other property to the drug. Once the chemical group has been cleaved from the prodrug the more active drug is generated. Prodrugs may be designed as reversible drug derivatives and utilized as modifiers to enhance drug transport to site-specific tissues. The design of prodrugs to date has been to increase the effective water solubility of the therapeutic compound for targeting to regions where water is the principal solvent. For example, Fedorak, et al., Am. J. Physiol, 269:G210-218 (1995), describe dexamethasone- beta -D-glucuronide. McLoed, et al., Gastroenterol., 106:405-413 (1994), describe dexamethasone-succinate-dextrans. Hochhaus, et al., Biomed. Chrom., 6:283-286 (1992), describe dexamethasone-21-sulphobenzoate sodium and dexamethasone-21-isonicotinate. Additionally, J. Larsen and H. Bundgaard [Int. J. Pharmaceutics, 37, 87 (1987)] describe the evaluation of N-acylsulfonamides as potential prodrug derivatives. J. Larsen et al., [Int. J. Pharmaceutics, 47, 103 (1988)] describe the evaluation of N-methylsulfonamides as potential prodrug derivatives. Prodrugs are also described in, for example, Sinkula et al., J. Pharm. Sci., 64:181-210 (1975).

The term "derivative" refers to a compound that is produced from another compound of similar structure by the replacement of substitution of one atom, molecule or group by another. For example, a hydrogen atom of a compound may be substituted by alkyl, acyl, amino, etc., to produce a derivative of that compound.

The phrase "pharmaceutically acceptable" is used adjectivally herein to mean that the modified noun is appropriate for use in a pharmaceutical product. Pharmaceutically acceptable cations include metallic ions and organic ions. More preferred metallic ions include, but are not limited to appropriate alkali metal salts, alkaline earth metal salts and other physiological acceptable metal ions. Exemplary ions include aluminum, calcium, lithium, magnesium, potassium, sodium and zinc in their usual valences. Preferred organic ions include protonated tertiary amines and quaternary ammonium cations, including in part, trimethylamine, diethylamine, N,N'-dibenzylethylenediamine, chloroprocaine, choline, diethanolamine, ethylenediamine, meglumine (N-methylglucamine) and procaine. Exemplary pharmaceutically acceptable acids include without limitation hydrochloric acid, hydrobromic acid, phosphoric acid, sulfuric acid, methanesulfonic acid, acetic acid, formic acid, tartaric acid, maleic acid, malic acid, citric acid, isocitric acid, succinic acid, lactic acid, gluconic acid, glucuronic acid, pyruvic acid oxalacetic acid, fumaric acid, propionic acid, aspartic acid, glutamic acid, benzoic acid, and the like.

The phrase "penetration enhancing agent" refers to an agent known to accelerate the delivery of the drug through the skin. These agents also have been referred to as accelerants, adjuvants, and absorption promoters, and are collectively referred to herein as "enhancers." This class of agents includes those with diverse mechanisms of action including those which have the function of improving the solubility and diffusibility of the drug, and those which improve percutaneous absorption by changing the ability of the stratum corneum to retain moisture, softening the skin, improving the skin's permeability, acting as penetration assistants or hair-follicle openers or changing the state of the skin such as the boundary layer. The penetration enhancing agent of the present invention is a functional derivative of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the -COOH group is substituted with a functional derivative thereof, such as alcohols, polyols, amides and substituted derivatives thereof. The term "fatty acid" means a fatty acid that has four (4) to twenty-four (24) carbon atoms.

Non-limiting examples of penetration enhancing agents include C₈-C₂₂ fatty acids such as isostearic acid, octanoic acid, and oleic acid; C₈-C₂₂ fatty alcohols such as oleyl alcohol and lauryl alcohol; lower alkyl esters of C₈-C₂₂ fatty acids such as ethyl oleate, isopropyl myristate, butyl stearate, and methyl laurate; di(lower)alkyl esters of C₆-C₂₂ diacids such as diisopropyl adipate; monoglycerides of C₈-C₂₂ fatty acids such as glyceryl monolaurate; tetrahydrofurfuryl alcohol; polyethylene glycol ether; polyethylene glycol; propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; N-alkylpyrrolidone; and terpenes.

The thickening agents used herein may include anionic polymers such as polyacrylic acid (CARBOPOL® by B.F. Goodrich Specialty Polymers and Chemicals Division of Cleveland, Ohio), carboxypolymethylene, carboxymethylcellulose and the like, including derivatives of Carbopol® polymers, such as Carbopol® Ultrez 10, Carbopol® 940, Carbopol® 941, Carbopol® 954, Carbopol® 980, Carbopol® 981, Carbopol® ETD 2001, Carbopol® EZ-2 and Carbopol® EZ-3, and other polymers such as Pemulen® polymeric emulsifiers, and Noveon® polycarbophils. Additional thickening agents, enhancers and adjuvants may generally be found in Remington's The Science and Practice of Pharmacy, Meade Publishing Co., United States Pharmacopeia/National Formulary.

As used herein, the term "lower alcohol," alone or in combination, means a straight-chain or branched-chain alcohol moiety containing one to about six carbon atoms. In one embodiment, the lower alcohol contains one to about 4 carbon atoms, and in another embodiment the lower alcohol contains two to about 3 carbon atoms. Examples of such alcohol moieties include methanol, ethanol, n-propanol, isopropanol, n-butanol, isobutanol, sec-butanol, and tert-butanol.

As used herein, the term "lower alkyl", alone or in combination, means a straight-chain or branched-chain alkyl radical containing one to about six carbon atoms. In one embodiment, the lower alkyl contains one to about four carbon atoms. Examples of such radicals include methyl, ethyl, n-propyl, isopropyl, n-butyl, isobutyl, sec-butyl, and tert-butyl.

Decreased production of testosterone in a subject can be caused by several factors well known to those skilled in the relevant field of medicine.

Physiological and psychological disorders associated with testosterone deficiency in a subject include, for example, decreased mood, libido and sexual performance, decreased bone mineral density and related markers, diminished body composition, human immunodeficiency virus wasting syndrome, decreased cognition, diminished mood and self-esteem, decreased muscle mass and performance, premenstrual syndrome, and autoimmune disease.

Nevertheless, there exist well-defined subject populations where testosterone production is clearly deficient and where associated symptomatology has been described, and such populations are contemplated as falling within the scope of the present invention.

Subjects to be treated with the present invention include those currently experiencing a depressive disorder event. Standard depressive disorder risk factors are known to the average physician practicing in the relevant field of medicine.

In addition, contemplated methods and compositions of the present invention are useful to treat testosterone deficiency in a subject, which includes a subject where testosterone production is deficient, or where the associated symptomatology related to deficient testosterone production is clinically evident. In men, this includes age, for example.

In an embodiment of the present invention, the methods and composition are useful in treating a subject who has decreased adrenal function. Decreased adrenal function, which may result from a variety of causes, represents another category of subjects where testosterone production may be reduced by approximately 50%. Primary adrenocortical deficiency, or Addison's disease, is a rare endocrine disorder with multiple etiologies, including tuberculosis and fungal infections.

In one embodiment of the present invention, the methods and composition are useful in treating a subject where chronic corticosteroid therapy is administered. Chronic corticosteroid therapy is used for a variety of conditions, which include rheumatoid arthritis, systemic lupus erythematosus, Sjogren's syndrome, immunosuppression for transplants, asthma, etc. Corticosteroid-induced adrenal suppression may thus represent the largest group of subjects with deficient adrenal androgen production. Androgen deficiency is recognized as a contributory factor to corticosteroid-induced osteoporosis. In a subject with autoimmune disorders, such as rheumatoid arthritis and systemic lupus erythematosus, testosterone deficiency can contribute to the underlying tendency to produce autoantibodies, as has been seen in a variety of animal models of autoimmune disease. Testosterone replacement can thus help to ameliorate the autoimmune disease process, itself. Despite these considerations, the potential therapeutic benefits of testosterone replacement in treating corticosteroid suppressed subjects have largely been ignored.

In yet another embodiment of the present invention, the methods and composition are useful in treating a subject with primary adrenal insufficiency. Primary adrenal insufficiency is associated with testosterone deficiency.

In one embodiment of the present invention, the methods and composition are useful in treating a subject with chronic illnesses. Chronic illnesses in a subject are attended by decreased circulating testosterone concentrations. Glucocorticoid administration inhibits adrenal androgen production by their inhibitory effects on adrenocorticotropic hormone secretion. In addition, glucocorticoids also have inhibitory effects at all levels of the hypothalamic-pituitary-ovarian axis.

In still another embodiment of the present invention, the methods and composition are useful in treating a human immunodeficiency virus-positive man. In contrast to human immunodeficiency virus-positive men, where testosterone deficiency is common, it is not known whether human immunodeficiency virus-positive women are deficient in testosterone. Adrenal function can also be deficient in acquired immunodeficiency syndrome subjects due to cytomegalovirus infection, tuberculosis and/or fungal infections. Megestrol acetate, a progestational agent used to stimulate appetite in human immunodeficiency virus infected persons, suppresses gonadotropins and is it believed to lower testosterone levels.

In one embodiment of the present invention, the methods, and compositions are useful in suppressing both cell-mediated and humoral immune responses in a subject. Androgens appear to suppress both cell-mediated and humoral immune responses. Many researchers have advocated increasing testosterone levels in a subject as protective against autoimmune disease, such as rheumatoid arthritis. Testosterone administration therefore is contemplated to be effective in treating a subject with such disorders.

Toxicity and therapeutic efficacy of the therapeutic agents of the present invention can be determined by standard pharmaceutical procedures, for example, for determining LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The active agents of the present invention may be administered, if desired, in the form of a salt, an ester, an amide, an enantiomer, an isomer, a tautomers, a prodrug, a derivative or the like, provided the salt, ester, amide, enantiomer, isomer, tautomer, prodrug, or derivative is suitable pharmacologically, that is, effective in the present methods, kits, combinations, and compositions. Salts, esters, amides, enantiomers, isomers, tautomers, prodrugs and other derivatives of the active agents may be prepared using standard procedures known to those skilled in the art of synthetic organic chemistry and described, for example, by J. March, Advanced Organic Chemistry; Reactions, Mechanisms and Structure, 4th Ed. (New York: Wiley-Interscience, 1992). For example, acid addition salts are prepared from the free base using conventional methodology, and involves reaction with a suitable acid. Generally, the base form of the drug is dissolved in a polar organic solvent such as methanol or ethanol and the acid is added thereto. The resulting salt either precipitates or may be brought out of solution by addition of a less polar solvent. Suitable acids for preparing acid addition salts include both organic acids, for example, acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, for example, hydrochloric acid, hydrobromic acid, sulfuric acid, nitric acid, phosphoric acid, and the like. An acid addition salt may be reconverted to the free base by treatment with a suitable base. Particularly preferred acid addition salts of the active agents herein are halide salts, such as may be prepared using hydrochloric or hydrobromic acids. Particularly preferred basic salts here are alkali metal salts, for example, the sodium salt, and copper salts. Preparation of esters involves functionalization of hydroxyl and/or carboxyl groups which may be present within the molecular structure of the drug. The esters are typically acyl-substituted derivatives of free alcohol groups, that is, moieties that are derived from carboxylic acids of the formula RCOOH where R is alkyl, and preferably is lower alkyl. Esters can be reconverted to the free acids, if desired, by using conventional hydrogenolysis or hydrolysis procedures. Amides and prodrugs may also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine. Prodrugs are typically prepared by covalent attachment of a moiety, which results in a compound that is therapeutically inactive until modified by an individual's metabolic system.

The therapeutic agents for use in accordance with the present invention can be formulated as a single pharmaceutical composition containing at least one therapeutic agent, for example, testosterone alone or with an antidepressant agent, or as independent multiple pharmaceutical compositions where each composition contains at least one therapeutic agent. Pharmaceutical compositions according to the present invention include those compositions with at least one therapeutic agent formulated for percutaneous administration. Percutaneous administration includes transdermal delivery systems that include patches, gels, tapes and creams, and can contain excipients such as alcohols, penetration enhancing agents, hydroxide releasing agents, and thickening agents, as well as solubilizers (for example propylene glycol, bile salts, and amino acids), hydrophilic polymers (for example, polycarbophil and polyvinylpyrolidone), and adhesives and tackifiers (for example, polyisobutylenes, silicone-based adhesives, acrylates and polybutene).

The therapeutic agents for use in accordance with the present invention can then be administered percutaneously in dosage unit formulations containing conventional nontoxic pharmaceutically acceptable carriers, adjuvants, and vehicles as desired.

The compounds for use in accordance with the present invention can be administered by any conventional means available for use in conjunction with pharmaceuticals, either as individual therapeutic compounds or as a combination of therapeutic compounds.

The compositions of the present invention can be administered for treating, preventing, or reducing the risk of developing a testosterone deficiency in a subject by any means that produce contact of these compounds with their site of action in the body, for example in the ileum, the plasma, or the liver of a subject. For example the compositions can be administered, for example, orally, rectally, topically, bucally, or parenterlly.

Additionally, the compositions of the present invention may optionally include salts, emollients, stabilizers, antimicrobials, fragrances, and propellants.

In another embodiment of the present invention, the therapeutic agents come in the form of kits or packages containing testosterone. Illustratively, the kits or packages contain testosterone in a dosage form suitable for percutaneous administration, for example, a gel, a cream, an ointment, or a patch, in amounts for the proper dosing of the drugs. The therapeutic agents for use in accordance with the present invention can be packaged in the form of kits or packages in which the daily (or other periodic) dosages are arranged for proper sequential or simultaneous administration. The present invention further provides a kit or package containing a plurality of dosage units, adapted for successive daily administration, each dosage unit comprising at least one of the therapeutic agents for use in accordance with the present invention. This drug delivery system can be used to facilitate administering any of the various embodiments of the therapeutic compositions. In one embodiment, the system contains a plurality of dosages to be to be administered daily or weekly where at least one of the dosages is administered via percutaneous administration. In another embodiment, the system contains a plurality of dosages to be to be administered daily or weekly where at least one of the dosages is administered via percutaneous administration, and at least one of the dosages is administered orally. The kits or packages also contain a set of instructions for the subject.

The present methods and compositions are used in "combination therapy" with an antidepressant agent.

The phrase "combination therapy" embraces the administration of a steroid in the testosterone synthesis pathway in conjunction with an antidepressant agent, as part of a specific treatment regimen intended to provide a beneficial effect from the co-action of these therapeutic agents for the treatment of a depressive disorder in a subject. The beneficial effect of the combination includes, but is not limited to, pharmacokinetic or pharmacodynamic co-action resulting from the combination of therapeutic agents. Administration of these therapeutic agents in combination typically is carried out over a defined time period (usually simultaneously, minutes, hours, days, weeks, months or years depending upon the combination selected). "Combination therapy" generally is not intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, where each therapeutic agent is administered at a different time, as well as administration of these therapeutic agents, or at least two of the therapeutic agents, in a substantially simultaneous manner. Substantially simultaneous administration can be accomplished, for example, by administering to the subject a single gel having a fixed ratio of each therapeutic agent or in multiple, single capsules, tablets, or gels for each of the therapeutic agents. Sequential or substantially simultaneous administration of each therapeutic agent can be effected by any appropriate route including, but not limited to, an oral route, a percutaneous route, an intravenous route, an intramuscular route, or by direct absorption through mucous membrane tissues. The therapeutic agents can be administered by the same route or by different routes. For example, a first therapeutic agent of the combination selected may be administered orally, while the other therapeutic agents of the combination may be administered percutaneously. Alternatively, for example, all therapeutic agents may be administered percutaneously, or all therapeutic agents may be administered intravenously, or all therapeutic agents may be administered intramuscularly, or all therapeutic agents can be administered by direct absorption through mucous membrane tissues. The sequence in which the therapeutic agents are administered is not narrowly critical. "Combination therapy" also can embrace the administration of the therapeutic agents as described above in further combination with other biologically active ingredients, such as, but not limited to, agents for improving sexual performance, such as, for example, an agent effective at inhibiting the activity of a phosphodiesterase, and non-drug therapies, such as, but not limited to, surgery.

The therapeutic compounds which make up the combination therapy may be a combined dosage form or in separate dosage forms intended for substantially simultaneous administration. The therapeutic compounds that make up the combination therapy may also be administered sequentially, with either therapeutic compound being administered by a regimen calling for two step administration. Thus, a regimen may call for sequential administration of the therapeutic compounds with spaced-apart administration of the separate, active agents. The time period between the multiple administration steps may range from, for example, a few minutes to several hours to days, depending upon the properties of each therapeutic compound such as potency, solubility, bioavailability, plasma half-life and kinetic profile of the therapeutic compound, as well as depending upon the effect of food ingestion and the age and condition of the subject. Circadian variation of the target molecule concentration may also determine the optimal dose interval. The therapeutic compounds of the combined therapy whether administered simultaneously, substantially simultaneously, or sequentially, may involve a regimen calling for administration of one therapeutic compound by oral route and another therapeutic compound by percutaneous route. Whether the therapeutic compounds of the combined therapy are administered orally, by inhalation spray, rectally, topically, buccally (e.g., sublingual), or parenterally (e.g., subcutaneous, intramuscular, intravenous and intradermal injections, or infusion techniques), separately or together, each such therapeutic compound will be contained in a suitable pharmaceutical formulation of pharmaceutically-acceptable excipients, diluents or other formulations components. Examples of suitable pharmaceutically-acceptable formulations containing the therapeutic compounds are given above. Additionally, drug formulations are discussed in, for example, Hoover, John E., Remington's Pharmaceutical Sciences, Mack Publishing Co., Easton, Pennsylvania 1975. Another discussion of drug formulations can be found in Liberman, H.A. and Lachman, L., Eds., Pharmaceutical Dosage Forms, Marcel Decker, New York, N.Y., 1980.

The present invention is further illustrated by the following examples, which should not be construed as limiting in any way.

The following examples are provided for exemplification of the present invention and are not intended to be limiting in any way.

### EXAMPLES

### Example 1: Method of Treating a Depressive Disorder in a Subject

An eight-week randomized placebo-controlled trial of testosterone transdermal gel (AndroGel®) was conducted with 22 treatment-refractory depressed men with low or borderline total testosterone levels (≤ 350 ng/dl). Testosterone gel, added to the subjects' existing antidepressant regimens, proved significantly superior to placebo in antidepressant response on the HAM-D and the CGI-severity scales, although not on the BDI.

Men age 30-65 years, presently taking an adequate dose of antidepressant medication (as defined by the manufacturer's published product information) for at least the last four weeks, but still complaining of depressive symptoms sufficient to meet DSM-IV criteria for current major depressive disorder. Subjects were initially screened. The screening was scheduled so as to have testosterone at the diurnal maximum level (prior to 10am). Subjects were then administered the depression module the Structured Clinical Interview for DSM-IV (SCID) to confirm the diagnosis of current major depressive disorder. Subjects were next administered the American Urological Association (AUA) Symptom Index for benign prostatic hyperplasia (BPH), subjects scoring higher than 14 on this index were excluded. Blood was then collected for total testosterone and PSA levels.

Men who displayed low or borderline morning testosterone levels (100-350 ng/dl; normal range, 270-1070 ng/dl) and normal PSA levels (< 1.5 ng/ml in men age 30-39, < 2.5 ng/ml in men 40-49, < 3.5 ng/ml in men 50-59, and < 4.0 ng/ml in men 60-64) were chosen for a second screening evaluation. Next, the subjects were administered: 1) basic demographic questions; 2) the remainder of the SCID; 3) questions regarding history of previous antidepressant drug treatment; 4) the HAM-D; 5) the BDI; 6) the Clinical Global Impression Scale (CGI); 7) medical history questions; 8) physical examination, including vital signs, height, weight, and digital rectal examination of the prostate; 9) laboratory tests for standard chemistries, hematology, urinalysis, and HIV serology; 10) electrocardiogram (EKG); and 11) determination of body fat with calipers, together with calculation of fat-free mass index (FFMI), a measure of muscularity previously developed in our laboratory. Subjects were excluded if they exhibited 1) any substance use disorder within the past year (or illicit anabolic steroid use at any time in their lives); 2) current or past psychotic symptoms; 3) a history of bipolar disorder; 4) any abnormality on digital rectal examination; or 5) evidence of other clinically significant medical disease on the basis of medical history and physical examination.

Qualifying subjects were then started on a one-week single-blind placebo washout with placebo gel. All subjects were asked to continue taking their existing antidepressant medications, together with any other medications that they were prescribed, at their present dose throughout the study.

Baseline (Week 0): Subjects were assessed for scores on the HAM-D, BDI, and CGI-Severity of Illness; adverse events; and vital signs. Results from the laboratory tests drawn at screen and from EKG readings were also reviewed. Subjects were eliminated if they: a) displayed more than 50% improvement on the HAM-D or BDI after the placebo treatment; or b) were found to have a clinically significant abnormality on the laboratory tests or EKG. Subjects were then randomized to receive either 10 grams of 1% testosterone gel or placebo daily for 7 days. Drug and placebo were supplied in identical-appearing packets that contained either 2.5 g of AndroGel or a placebo gel.

Week 1: Subjects were assessed for scores on the HAM-D, BDI, and CGI (both Severity of Illness and Improvement as compared to Baseline); adverse events; and vital signs. Subjects provided blood for a total testosterone level, drawn at least four hours after the morning application of the gel.

Weeks 2, 4, 6, and 8: Subjects were assessed at weeks 2, 4,6 and 8 for HAM-D, BDI, CGI, adverse events, and vital signs. Week 8, subjects received an additional determination of PSA and measurement of weight and body fat. The blind was then broken and the correct identity of treatment assignment determined.

Subjects were eliminated prior to week 8 if they: 1) voluntarily elected to withdraw for any reason; 2) displayed an adverse event judged clinically significant by the investigators; or 3) failed to comply with the requirements of the protocol.

Statistical Analysis: Baseline characteristics of each group were compared using Fisher's exact test for categorical variables and the *t*-test for continuous variables. Two populations of patients were defined: (1) an intent-to-treat group of patients with at least one available efficacy measure, and (2) a completers group, defined as patients who completed the 8-week treatment period.

The primary protocol-defined analysis of efficacy was a repeated measures random regression analysis comparing the rate of change of scores on the HAM-D, BDI, and CGI-severity during the treatment period between groups, using methods described by Diggle et al. and Gibbons et al. A model was used for the mean of the outcome variable that included terms for treatment, time, and treatment-by-time interaction. Time as a continuous variable was modeled, with weeks ranging from 0 (Baseline) to 8 (after randomization). The measure of effect was the treatment-by-time interaction (or the difference in the rate of change per unit of time, or the difference in slope with respect to time) of the efficacy measure. To account for the correlation of observations within individuals, the standard errors of the parameter estimates were calculated using generalized estimating equations, with compound symmetry as the working covariance, as implemented by the PROC GENMOD command in SAS software.

As secondary analyses of the outcome measures, two analyses of change from baseline to endpoint were used: 1) an intent-to-treat analysis, using the last observation carried forward for all subjects completing at least one post-baseline assessment; and 2) a completers analysis, using all subjects who completed 8 weeks of randomized treatment. The *t*-test was used to compare the difference between groups in change from baseline to endpoint on the HAM-D, BDI, and CGI-severity.

For laboratory measures, including body fat and FFMI, the mean difference between endpoint and baseline measures were used, and then compared the treatment groups using the *t*-test. The correlation coefficients were calculated by using rank-transformed data (Spearman rank correlation). All statistical tests were two-sided with alpha=0.05.

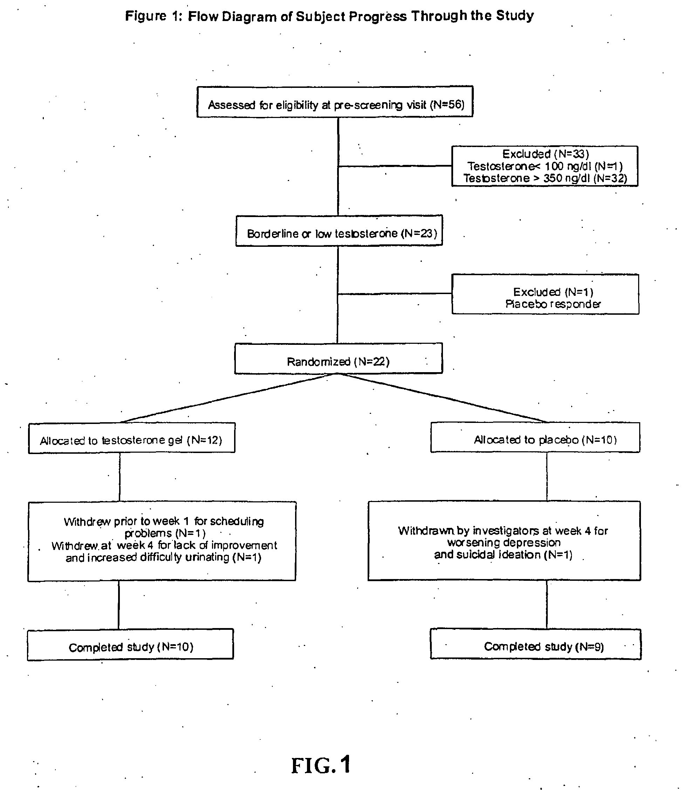

Recruitment and participant flow: The mean (SD) age of the subjects was 46.9 (9.2) years (range 30-65); all 56 subjects met the PSA and BPH criteria for the study described above. The men's total testosterone levels, despite being measured near their diurnal maximum, were remarkably low for their age range (1.27), with a median (interquartile range) of only 376 (301,477) ng/dl. Total testosterone levels were inversely correlated with age, but only weakly so (Spearman *ρ* = -0.25; P = 0.06). Twenty-four (43.6%) of the subjects displayed levels of 350 ng/dl or less. Their median baseline total testosterone level was 292 (266,309) ng/dl. All of the remaining 22 subjects were randomized at Week 0. Of these, 3 (14%) withdrew during the follow-up period and 19 (86%) completed the full 8 weeks of the study. (Figure No. 1)

**Table 7**

| **Demographic and Clinical Characteristics of Subjects at Screen** | | |
|---|---|---|
| Characteristic | Randomized to Testosterone (N=12) | Randomized to Placebo (N=10) |
| | N | N |
| Ethnicity | | |
| Caucasian | 11 | 10 |
| African-American | 1 | 0 |

| Marital Status | | |
|---|---|---|
| Married | 8 | 8 |
| Single | 2 | 1 |
| Divorced | 2 | 1 |

| Sexual Orientation | | |
|---|---|---|
| Heterosexual | 11 | 10 |
| Homosexual | 1 | 0 |

| | Mean | Mean |
|---|---|---|
| Age (years) | 48.9 | 49,5 |
| Height (cm) | 177 | 181 |
| Weight (kg) | 93.3 | 104.5 |
| Body Fat Percentage | 28.5 | 30.4 |
| Fat-Free Mass Index (kg/m2) | 21.2 | 22 |
| Prostate-Specific Antigen (ng/ml) | 0.8 | 0.8 |
| Total Testosterone Level (ng/dl) | 293 | 267 |
| Hamilton Depression Rating Scale^{b} | 21.8 | 21.3 |
| Beck Depression Inventory^{b} | 23.1 | 23.6 |
| Clinical Global Impression - Severity^{b} | 4.7 | 4.3 |

| | | |
|---|---|---|
| Represents score at baseline; all other variables are at screen | | |

Baseline characteristics of subjects: The 12 subjects randomized to testosterone did not differ significantly from the 10 randomized to placebo on attributes at screen (Table 7 ), except that the placebo subjects were slightly heavier than testosterone subjects. The antidepressant regimens of the subjects were SSRI's (5 testosterone subjects, 8 placebo subjects), bupropion (2 testosterone), bupropion plus SSRI's (2 placebo), venlafaxine (3 testosterone), nefazodone (1 testosterone), and methylphenidate (1 testosterone).

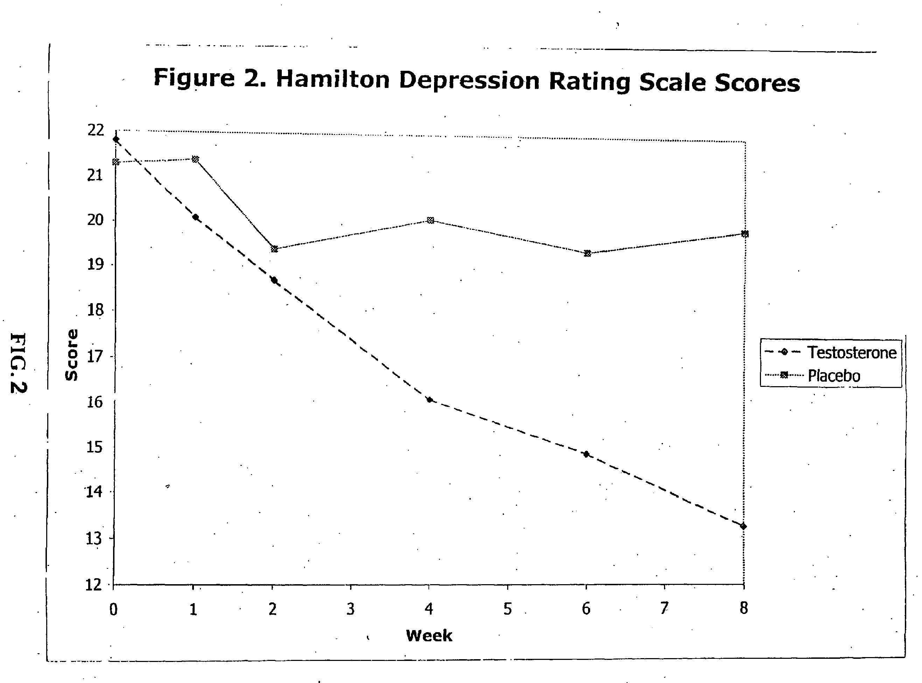

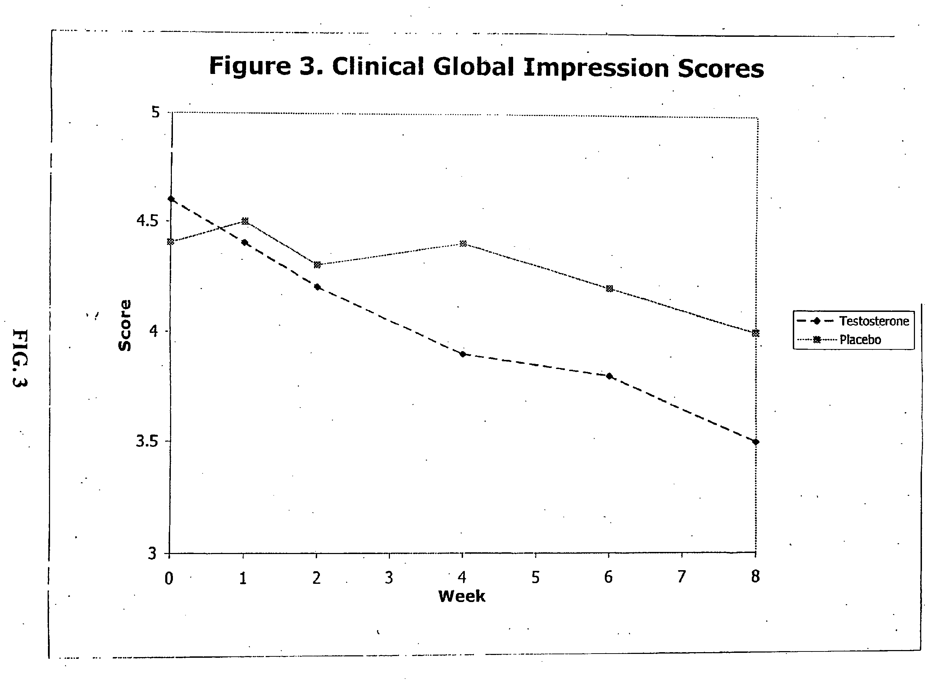

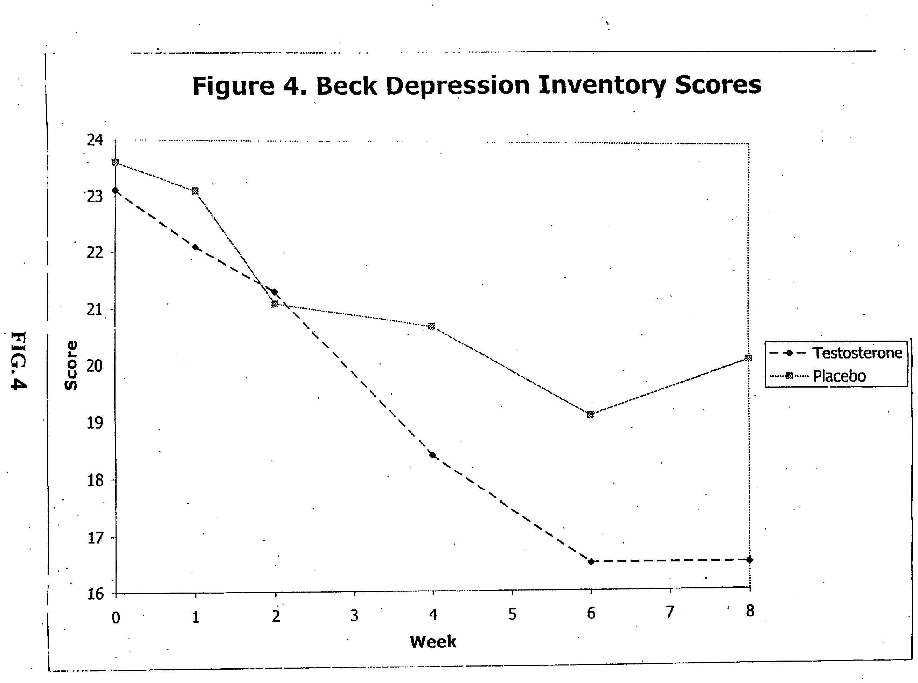

Efficacy analyses: The primary analysis of efficacy, involving all 22 subjects with at least one rating of outcome measures, revealed that testosterone-treated patients had a significantly greater rate of decrease in HAM-D scores than placebo-treated patients (Figure No. 2). This improvement was evident on both the vegetative and affective symptoms subscales of the HAM-D (Table 8). Testosterone was also associated with significantly greater rates of decrease in CGI-severity scores (Figure No. 3), although not BDI scores (Figure No. 4). All rate-of-change data are summarized in Table 8 . The endpoint analyses produced similar results, but with slightly less statistical power than the longitudinal analysis .

**Table 8**

| Mean change (SD) on outcome measures from baseline to endpoint, by treatment group | | | | |
|---|---|---|---|---|
| | **Intent to Treat^{a}** | | **Completers^{b}** | |
| | **Placebo** | **Testosterone** | **Placebo** | **Testosterone** |
| Outcome Measure | **N=10** | **N=11** | **N=9** | **N=10** |
| HAM-D, Total score | -0.3 (4.0) | -7.4 (7.1) | -1.1 (3.2) | -8.8 (6.0) |
| HAM-D, Affective Subscale | 0.0 (1.5) | -2.1 (3.4) | -0.2 (1.4) | -2.7 (2.9) |
| HAM-D, Vegetative Subscale | -0.7 (2.5) | -3.2 (2.0) | -0.9 (2.5) | -3.5 (1.8) |
| BDI, Total Score | -2.0 (5.2) | -5.5 (8.7) | -2.4 (5.3) | -6.8 (7.8) |
| CGI -Severity | -0.2 (0.6) | -0.9 (1.4) | -0.3 (0.5) | -1.2 (1.0) |
| CGI-Improvement | 3.90 (0.88) | 3.09 (1.14) | 3.67 (0.50) | 2.9 (0.99) |

| | | | | |
|---|---|---|---|---|
| a- Last observation carried forward as endpoint; includes all subjects who completed at least one post-baseline visit b- Week 8 as endpoint | | | | |

Among study completers, there were no significant differences between subjects receiving testosterone and those receiving placebo on change in percent body fat [-2.8 (1.7)% vs. -1.9 (2.6)%; t = 0.90, df = 17, p = 0.38] or change in muscle mass as expressed by FFMI [1.1 (0.9) vs. 0.6 (1.2) kg/meter²; t = 1.03, df = 17, p = 0.32].

Mean testosterone levels at Week 1 were 789 (519) ng/dl in the testosterone group vs. 249 (68) ng/dl in the placebo group (t=3.26, df = 19, p = 0.004). Notably, 3 of 11 testosterone subjects displayed ≤ 70 ng/dl increase in their total testosterone levels with the gel; these same subjects also displayed little improvement in depressive symptoms (changes of 0,0, and 1, respectively on CGI-severity at termination). The remaining 8 subjects all achieved ≥ 200 ng/dl increase in testosterone levels at Week 1; 4 (50%) of these subjects improved by 2 points or more on CGI-severity, as compared to none of the 10 subjects receiving placebo (p = 0.023 by Fisher's exact test, two-tailed). Testosterone gel benefited psychological aspects of depression (such as the depressed mood, guilt, and psychological anxiety items on the HAM-D) to nearly the same degree as the somatic aspects of depression (such as the HAM-D items involving sleep, appetite, libido, and somatic symptoms). Preliminary data suggest that in much lower doses, testosterone may exhibit antidepressant effects in women as well.

The practice of the present invention will employ, unless otherwise indicated, conventional techniques of pharmacology and pharmaceutics, which are within the skill of the art.

## Claims

1. A pharmaceutical composition for use in a method of treating a depressive disorder in a subject that has failed to respond to conventional antidepressants, said composition comprising:
(a) 0.01% to 70% of testosterone;
(b) 0.01% to 50% of penetration enhancing agent;
(c) 0.01% to 50% of thickening agent; and
(d) 30% to 98% of straight-chain or branched-chain alcohol moiety containing one to six carbon atoms;
the percentages being a weight to weight basis of the composition;
wherein the subject is a man;
wherein the composition is capable of releasing testosterone after applying the composition to the skin at a rate and duration that delivers at least 10 µg per day of testosterone to the blood serum of the subject; and
wherein the composition is used in conjunction with an antidepressant agent and wherein the amount of the composition and the amount of the therapeutic agent together make a depressive-disorder-effective amount.

2. The composition for use of claim 1, comprising 0.1% to 10% of testosterone, preferably 1% of testosterone.

3. The composition for use of claim 1, wherein the penetration enhancing agent comprises 0.1% to 5% of isostearic acid, octanoic acid, lauryl alcohol, ethyl oleate, isopropyl myristate, butyl stearate, methyl laurate, diisopropyl adipate, glyceryl monolaurate, tetrahydrofurfuryl alcohol, polyethylene glycol ether, polyethylene glycol, propylene glycol, 2-(2-ethoxyethoxy) ethanol, diethylene glycol monomethyl ether, alkylaryl ethers of polyethylene oxide, polyethylene oxide monomethyl ethers, polyethylene oxide dimethyl ethers, dimethyl sulfoxide, glycerol, ethyl acetate, acetoacetic ester, N-alkylpyrrolidone, or terpene.

4. The composition for use of claim 1, wherein the penetration enhancing agent is isopropyl myristate.

5. The composition for use of claim 1, wherein the thickening agent comprises 0.1 % to 5% polyacrylic acid, preferably 0.9% polyacrylic acid.

6. The composition for use of claim 5, wherein the polyacrylic acid is carboxypolymethylene.

7. The composition for use of claim 1, wherein the straight-chain or branched-chain alcohol comprises 45% to 90% ethanol or isopropanol.

8. The composition for use of claim 1, wherein, after applying the composition to the skin, the circulating serum concentration of the testosterone is greater than 400 ng testosterone per dl serum during a time period beginning 2 hours after administration and ending 24 hours after administration.

9. The composition for use of claim 8, wherein the serum testosterone concentration is between 400 ng testosterone per dl serum to 1050 ng testosterone per dl serum.

10. The composition for use of claim 1, wherein the composition is provided to the subject for daily administration in a 0.1 g to a 10 g dose.

11. The composition for use of claim 1, wherein the composition is provided as a separate component to a kit.

12. The composition for use of claim 1, wherein the subject has a pretreatment serum testosterone concentration less than 300 ng/dl and wherein after at least 30 days of daily administration serum testosterone concentration in the subject is at least 490 ng/dl to 860 ng/dl.

13. The composition for use of claim 1, wherein the composition is administered once, twice, or three times daily for at least 7 days.

## Patentansprüche

1. Pharmazeutische Zusammensetzung zur Verwendung in einem Verfahren zum Behandeln einer depressiven Störung in einem Subjekt, das nicht auf herkömmliche Antidepressiva angesprochen hat, wobei die Zusammensetzung umfasst:
(a) 0,01% bis 70% Testosteron;
(b)0,01% bis 50% eines penetrationsverbessernden Mittels;
(c) 0,01% bis 50% Verdickungsmittel; und
(d)30% bis 98% einer geradkettigen oder verzweigtkettigen Alkoholeinheit, die ein bis sechs Kohlenstoffatome enthält;
wobei die Prozentzahlen eine Gewicht-zu-Gewicht-Basis der Zusammensetzung sind;
wobei das Subjekt ein Mann ist;
wobei die Zusammensetzung nach dem Auftragen der Zusammensetzung auf die Haut mit einer Geschwindigkeit und Dauer, die mindestens 10 µg Testosteron pro Tag an das Blutserum des Subjekts abgibt, zum Freisetzen von Testosteron fähig ist; und
wobei die Zusammensetzung in Verbindung mit einem Antidepressivum verwendet wird, und wobei die Menge der Zusammensetzung und die Menge des Therapeutikums zusammen eine für eine depressive Störung wirksame Menge ergeben.

2. Zusammensetzung zur Verwendung nach Anspruch 1, die 0,1% bis 10% Testosteron, bevorzugt 1% Testosteron umfasst.

3. Zusammensetzung zur Verwendung nach Anspruch 1, wobei das penetrationsverbessernde Mittel 0,1% bis 5% Isostearinsäure, Octansäure, Laurylalkohol, Ethyloleat, Isopropylmyristat, Butylstearat, Methyllaurat, Diisopropyladipat, Glycerylmonolaurat, Tetrahydrofurfurylalkohol, Polyethylenglycolether, Polyethylenglycol, Propylenglycol, 2-(2-Ethoxyethoxy)ethanol, Diethylenglycolmonomethylether, Alkylarylether von Polyethylenoxid, Polyethylenoxidmonomethylether, Polyethylenoxiddimethylether, Dimethylsulfoxid, Glycerin, Ethylacetat, Acetessigsäureester, N-Alkylpyrrolidon oder Terpen umfasst.

4. Zusammensetzung zur Verwendung nach Anspruch 1, wobei das penetrationsverbessernde Mittel Isopropylmyristat ist.

5. Zusammensetzung zur Verwendung nach Anspruch 1, wobei das Verdickungsmittel 0,1 % bis 5% Polyacrylsäure, bevorzugt 0,9% Polyacrylsäure umfasst.

6. Zusammensetzung zur Verwendung nach Anspruch 5, wobei die Polyacrylsäure Carboxypolymethylen ist.

7. Zusammensetzung zur Verwendung nach Anspruch 1, wobei der geradkettige oder verzweigtkettige Alkohol 45% bis 90% Ethanol oder Isopropanol umfasst.

8. Zusammensetzung zur Verwendung nach Anspruch 1, wobei nach dem Auftragen der Zusammensetzung auf die Haut die zirkulierende Serumkonzentration des Testosterons größer als 400 ng Testosteron pro dl Serum während eines Zeitraums ist, der 2 Stunden nach der Verabreichung beginnt und 24 Stunden nach der Verabreichung endet.

9. Zusammensetzung zur Verwendung nach Anspruch 8, wobei die Serum-Testosteronkonzentration zwischen 400 ng Testosteron pro dl Serum bis 1050 ng Testosteron pro dl Serum liegt.

10. Zusammensetzung zur Verwendung nach Anspruch 1, wobei die Zusammensetzung dem Subjekt zur täglichen Verabreichung in einer 0,1 g- bis zu einer 10 g-Dosis bereitgestellt wird.

11. Zusammensetzung zur Verwendung nach Anspruch 1, wobei die Zusammensetzung als separater Bestandteil zu einem Kit bereitgestellt wird.

12. Zusammensetzung zur Verwendung nach Anspruch 1, wobei das Subjekt eine Serum-Testosteronkonzentration vor Behandlung von weniger als 300 ng/dl aufweist, und wobei nach mindestens 30 Tagen täglicher Verabreichung die Serum-Testosteronkonzentration im Subjekt mindestens 490 ng/dl bis 860 ng/dl beträgt.

13. Zusammensetzung zur Verwendung nach Anspruch 1, wobei die Zusammensetzung einmal, zweimal oder dreimal täglich für mindestens 7 Tage verabreicht wird.

## Revendications

1. Composition pharmaceutique destinée à être utilisée dans une méthode de traitement d'un trouble dépressif chez un sujet qui n'a pas répondu aux antidépresseurs classiques, ladite composition comprenant :
(a) 0,01 % à 70 % de testostérone ;
(b) 0,01 % à 50 % d'agent améliorant la pénétration ;
(c) 0,01 % à 50 % d'agent épaississant ; et
(d) 30 % à 98 % de fraction alcool à chaîne droite ou à chaîne ramifiée contenant 1 à 6 atomes de carbone ;
les pourcentages étant une base de poids sur poids de la composition ;
dans lequel le sujet est un homme ;
dans laquelle la composition est capable de libérer de la testostérone après application de la composition sur la peau à un débit et une durée permettant de délivrer au moins 10 µg par jour de testostérone au sérum sanguin du sujet ; et
dans laquelle la composition est utilisée conjointement avec un agent antidépresseur et dans laquelle la quantité de la composition et la quantité de l'agent thérapeutique forment ensemble une quantité efficace contre les troubles dépressifs.

2. Composition à utiliser selon la revendication 1, comprenant 0,1 % à 10 % de testostérone, de préférence 1 % de testostérone.

3. Composition à utiliser selon la revendication 1, dans laquelle l'agent renforçant la pénétration comprend 0,1 % à 5 % d'acide isostéarique, d'acide octanoïque, d'alcool laurylique, d'oléate d'éthyle, de myristate d'isopropyle, de stéarate de butyle, de laurate de méthyle, d'adipate de diisopropyle, de monolaurate de glycéryle, d'alcool tétrahydrofurfylique, d'éther de polyéthylène glycol, de polyéthylène glycol, de propylène glycol, de 2-(2-éthoxyéthoxy)éthanol, d'éther monométhylique de diéthylène glycol, d'éthers alkylaryliques de polyéthylène oxyde, d'éthers monométhyliques de polyéthylène oxyde, d'éthers diméthyliques de polyéthylène oxyde, de sulfoxyde de diméthyle, de glycérol, d'acétate d'éthyle, d'ester acétoacétique, de N-alkylpyrrolidone ou de terpène.

4. Composition à utiliser selon la revendication 1, dans laquelle l'agent améliorant la pénétration est le myristate d'isopropyle.

5. Composition à utiliser selon la revendication 1, dans laquelle l'agent épaississant comprend 0,1 % à 5 % d'acide polyacrylique, de préférence de l'acide polyacrylique à 0,9 %.

6. Composition à utiliser selon la revendication 5, dans laquelle l'acide polyacrylique est un carboxypolyméthylène.

7. Composition à utiliser selon la revendication 1, dans laquelle l'alcool à chaîne droite ou à chaîne ramifiée comprend de 45 % à 90 % d'éthanol ou d'isopropanol.

8. Composition à utiliser selon la revendication 1, dans laquelle, après application de la composition sur la peau, la concentration sérique circulante de la testostérone est supérieure à 400 ng de testostérone par dl de sérum pendant une période commençant 2 heures après l'administration et se terminant 24 heures après l'administration.

9. Composition à utiliser selon la revendication 8, dans laquelle la concentration sérique de testostérone est comprise entre 400 ng de testostérone par dl de sérum et 1050 ng de testostérone par dl de sérum.

10. Composition à utiliser selon la revendication 1, dans laquelle la composition est fournie au sujet pour une administration quotidienne en une dose de 0,1 g à 10 g.

11. Composition à utiliser selon la revendication 1, dans laquelle la composition est fournie en tant que composant séparé d'un kit.

12. Composition à utiliser selon la revendication 1, dans laquelle le sujet a une concentration sérique de testostérone avant traitement inférieure à 300 ng/dl et dans laquelle, après au moins 30 jours d'administration quotidienne, la concentration sérique de testostérone chez le sujet est d'au moins 490 ng/dl à 860 ng/dl.

13. Composition à utiliser selon la revendication 1, dans laquelle la composition est administrée une fois, deux fois ou trois fois par jour pendant au moins 7 jours.
